(19) Europäisches Patentamt
European Patent Office
Office européen des brevets

(11) **EP 2 777 725 A2**

## (12) EUROPEAN PATENT APPLICATION

(43) Date of publication:
**17.09.2014 Bulletin 2014/38**

(51) Int Cl.:
*A61L 27/50* *(2006.01)*

(21) Application number: **14159395.4**

(22) Date of filing: **13.03.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.03.2013 US 201313826304**

(71) Applicant: **Titan Spine, LLC**
**Mequon, WI 53092 (US)**

(72) Inventors:
• **Ullrich, Peter F., Jr.**
**NEENAH, WI Wisconsin 54956 (US)**
• **Patterson, Chad J.**
**PORT WASHINGTON, WI Wisconsin 53074 (US)**
• **Schneider, M Jennifer M.**
**GERMANTOWN, WI Wisconsin 53022 (US)**

(74) Representative: **Spadaro, Marco et al**
**Cantaluppi & Partners**
**Viale della Tecnica, 205**
**00144 Roma (IT)**

(54) **Surface and subsurface chemistry of an integration surface**

(57)   An interbody spinal implant and a process of producing the implant. The implant includes an integration surface having a roughened surface topography as at least part of the top surface, bottom surface, or both surfaces. The integration surface comprises at least one or more of (a) a plurality of grains; (b) intergranular boundaries between the plurality of grains; and (c) unsatisfied chemical bonds. The integration surface may be produced by texturing a surface by chemical processes, mechanical processes, or both to provide the plurality of grains and intergranular boundaries and chemically etching the surface to provide unsatisfied chemical bonds. In addition, the plurality of grains and the intergranular boundaries may etch at different or non-uniform etch rates.

FIG. 2A

EP 2 777 725 A2

FIG. 2B

**Description**

TECHNICAL FIELD

The present invention relates generally to the chemistry of integration surfaces on interbody spinal implants.

BACKGROUND OF THE INVENTION

In the simplest terms, the spine is a column made of vertebrae and discs. The vertebrae provide the support and structure of the spine while the spinal discs, located between the vertebrae, act as cushions or "shock absorbers." These discs also contribute to the flexibility and motion of the spinal column. Over time, the discs may become diseased or infected, may develop deformities such as tears or cracks, or may simply lose structural integrity (e.g., the discs may bulge or flatten). Impaired discs can affect the anatomical functions of the vertebrae, due to the resultant lack of proper biomechanical support, and are often associated with chronic back pain.

Several surgical techniques have been developed to address spinal defects, such as disc degeneration and deformity. Spinal fusion has become a recognized surgical procedure for mitigating back pain by restoring biomechanical and anatomical integrity to the spine. Spinal fusion techniques involve the removal, or partial removal, of at least one intervertebral disc and preparation of the disc space for receiving an implant by shaping the exposed vertebral endplates. An implant is then inserted between the opposing endplates.

Spinal fusion procedures can be achieved using a posterior or an anterior approach, for example. Anterior interbody fusion procedures generally have the advantages of reduced operative times and reduced blood loss. Further, anterior procedures do not interfere with the posterior anatomic structure of the lumbar spine. Anterior procedures also minimize scarring within the spinal canal while still achieving improved fusion rates, which is advantageous from a structural and biomechanical perspective. These generally preferred anterior procedures are particularly advantageous in providing improved access to the disc space, and thus correspondingly better endplate preparation.

There are a number of problems, however, with traditional spinal implants including, but not limited to, improper seating of the implant, implant subsidence (defined as sinking or settling) into the softer cancellous bone of the vertebral body, poor biomechanical integrity of the endplates, damaging critical bone structures during or after implantation, and the like. In summary, at least ten, separate challenges can be identified as inherent in traditional anterior spinal fusion devices. Such challenges include: (1) end-plate preparation; (2) implant difficulty; (3) materials of construction; (4) implant expulsion; (5) implant subsidence; (6) insufficient room for bone graft; (7) stress shielding; (8) lack of implant incorporation with vertebral bone; (9) limitations on radiographic visualization; and (10) cost of manufacture and inventory.

Traditional implants may provide sharp teeth or the like on the top and bottom surface of an implant to attempt to stabilize or secure the implant. The problem is that teeth or other sharp features can result in increased loading in the joint space. The sharp teeth and increased loading remodel and degrade bone, which the present inventors have found actually leads to implant instability. In other words, implants having aggressive teeth or ridges can create pressure points and undesired bone remodeling providing instability and movement of the implant.

SUMMARY OF THE INVENTION

To meet this and other needs, and in view of its purposes, the present invention provides for an implant with one or more integration surfaces having a roughened surface topography, without teeth. For example, the integration surfaces having a roughened surface topography as described in this document facilitate osteointegration (e.g., formation of a direct structural and functional interface between the artificial implant and living bone or soft tissue) with the surrounding living bone. In addition, the specially designed surfaces facilitate attachment of osteoblasts and stimulate osteoblasts to mature and produce bone at higher rates than other surfaces. Thus, the roughened surface topography is specially designed, at the microscopic level, to interact with the tissues and stimulate their natural remodeling and growth, and at a larger scale, to perform the function of generating non-stressful friction, which when combined with a surgical technique that retains the most rigid cortical bone structures in the disc space, allows for a friction fit that does not abrade, chip, perforate, or compromise the critical endplate structures.

In one embodiment, the present invention provides an interbody spinal implant comprising a top surface, a bottom surface, opposing lateral sides, and opposing anterior and posterior portions. At least a portion of the top surface, the bottom surface, or both surfaces comprises an integration surface having a roughened surface topography, without sharp teeth that risk damage to bone structures, adapted to grip bone through friction generated when the implant is placed between two adjacent vertebrae and to inhibit migration of the implant. The integration surface comprises (a) a plurality of grains; (b) intergranular boundaries between the plurality of grains; and (c) unsatisfied chemical bonds.

The integration surface has a roughened surface topography. The integration surface may also have fusion and biologically active surface geometry, for example, in regular repeating patterns. The integration surface may include macro features, micro features, and nano features. For example, the features may include a repeating pattern of smooth shapes oriented in opposition to the biologic forces on the implant and to the insertion direction.

The integration surface includes a plurality of grains. The plurality of grains may comprise individual grains of titanium, for example, or an alloy of titanium (e.g., titanium, aluminum, and vanadium). The plurality of grains may be composed of a grain body, which is bordered by a grain edge. In addition, the plurality of grains may include intergranular boundaries between the individual grains. The plurality of grains may be structured, patterned, and oriented as will be recognized by one of ordinary skill in the art. For example, the grains may comprise hexagonal crystals. The plurality of grains may have an average diameter ranging from about 1-20 $\mu$m or from about 1-5 $\mu$m, for example.

The integration surface preferably includes a plurality of unsatisfied chemical bonds. These unsatisfied chemical bonds may include hydroxylated reactive groups, for example. In addition, the integration surface and the unsatisfied chemical bonds may be hydrophilic and/or hydrophobic in nature. The unsatisfied chemical bonds may be provided in a hydrated or carbonated environment to allow for more appropriate attachment of the organic materials to the integration surface.

The integration surface may include an outermost surface (e.g., the surface in direct contact with the vertebrae) and a subsurface (e.g., an area below the outermost surface). The subsurface may be non-uniform and certain portions of the subsurface may be exposed to the vertebrae. For example, the subsurface may be composed of the plurality of grains having unsatisfied bonds. In addition, the subsurface may exist at a given distance or depth into the bulk of the material. The subsurface may penetrate a distance of from about 1-5 $\mu$m, for example. In one embodiment, the subsurface exists at a distance or depth of about a single grain diameter.

According to another embodiment, the present invention provides a process for producing an integration surface on an interbody spinal implant having a top surface and a bottom surface where at least one of the top surface and the bottom surface comprises the integration surface having a roughened surface topography, without sharp teeth that risk damage to bone structures, adapted to grip bone through friction generated when the implant is placed between two vertebrae and to inhibit migration of the implant. The process may include chemically and/or mechanically texturing a surface (e.g., masked or unmasked chemical etching, masked or unmasked abrasive blasting, and the like) to provide a plurality of grains and intergranular boundaries between the plurality of grains. The surface may also be chemically etched (e.g., an acid etching) to provide unsatisfied chemical bonds. The plurality of grains and the intergranular boundaries may etch at different or non-uniform etch rates with respect to one another (e.g., the intergranular boundaries may etch at a faster rate than the grain bodies). The implant may be subsequently cleaned and packaged to preserve the grains, the intergranular boundaries, and the unsatisfied chemical bonds. For example, the packaging may include preserving the interbody spinal implant in an anaerobic environment.

The roughened surface topography may be fabricated, for example, using macro processing, micro processing, and nano processing techniques. The macro, micro, and nano process may include mechanical or chemical removal of at least a portion of the surface. For example, the macro features may be formed by heavy mechanical or chemical bulk removal, the micro features may be formed by mechanical or chemical removal, and the nano features may be formed by mild chemical etching, laser or other directed energy material removal, abrasion, blasting, or tumbling.

For example, the macro features may have a mean spacing between about 400-2,000 microns, a maximum peak-to-valley height between about 40-500 microns, and an average amplitude between about 20-200 microns; the micro features may have a mean spacing between about 20-400 microns, a maximum peak-to-valley height between about 2-40 microns, and an average amplitude between about 1-20 microns; and the nano features may have a mean spacing between about 0.5-20 microns, a maximum peak-to-valley height between about 0.2-2 microns, and an average amplitude between about 0.01-1 microns.

The implant may be fabricated from any suitable material. For example, the implant may be comprised of a metal, such as titanium. In the case of a composite implant (e.g., a body with one or more integration plates), the implant body may be fabricated from a non-metallic material, non-limiting examples of which include polyetherether-ketone, hedrocel, ultra-high molecular weight polyethylene, and combinations thereof. The implant body may be fabricated from both a metal and a non-metallic material to form a composite implant. For example, various types of ceramics may be used as biocompatible implant materials. Without restriction, titanium, alumina, zirconia, silicon nitrides, and others may be used as implant materials. For example, a composite implant may be formed with integration plates made of titanium combined with a polymeric body.

The implant may comprise a substantially hollow center and a vertical aperture. For example, the vertical aperture may (a) extend from the top surface to the bottom surface, (b) have a size and shape predetermined to maximize the surface area of the top surface and the bottom surface available proximate the anterior and posterior portions while maximizing both radiographic visualization and access to the substantially hollow center, and (c) define a transverse rim.

Various implant body shapes are provided to allow for implantation through various access paths to the spine through a patient's body. Certain embodiments of the present invention may be especially suited for placement between adjacent human vertebral bodies. The implants of the present invention may be used in procedures such as Anterior Lumbar Interbody Fusion (ALIF), Posterior Lumbar Interbody Fusion (PLIF), Transforaminal Lumbar Interbody Fusion (TLIF), and cervical fusion. Other applications of these treatments can be included in the production of implantable devices.

It is to be understood that both the foregoing general description and the following detailed description are exemplary, but are not restrictive, of the invention.

BRIEF DESCRIPTION OF THE DRAWING

The invention is best understood from the following detailed description when read in connection with the accompanying drawing. It is emphasized that, according to common practice, the various features of the drawing are not to scale. On the contrary, the dimensions of the various features are arbitrarily expanded or reduced for clarity. Included in the drawing are the following figures:

FIG. 1 shows a schematic cross-sectional representation of the material including the integration surface of the implant;

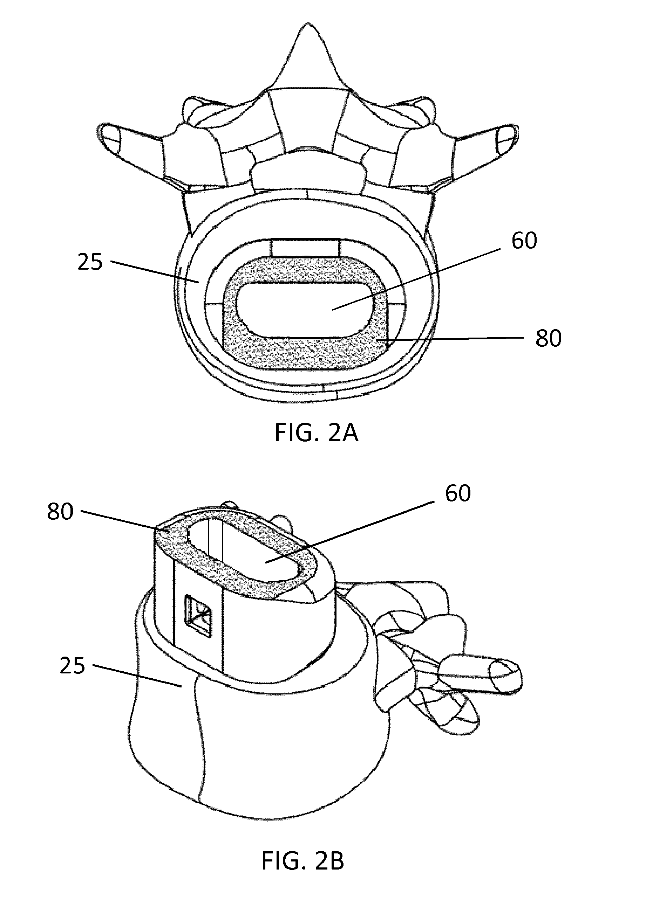

FIG. 2A shows a top view of an embodiment of the interbody spinal implant having a single vertical aperture and substantially hollow center positioned on a vertebral endplate;

FIG. 2B shows a perspective view of the embodiment of the interbody spinal implant illustrated in FIG. 2A;

FIG. 3A shows a perspective view of an embodiment of the interbody spinal implant having a generally oval shape and roughened surface topography on the top surface;

FIG. 3B shows a top view of the embodiment of the interbody spinal implant illustrated in FIG. 3A;

FIG. 4A shows a perspective view from the front of another embodiment of the interbody spinal implant according to the present invention;

FIG. 4B shows a perspective view from the rear of the embodiment of the interbody spinal implant illustrated in FIG. 4A;

FIG. 5A shows a perspective view from the front of another embodiment of the interbody spinal implant according to the present invention;

FIG. 5B is a top view of the interbody spinal implant illustrated in FIG. 5A;

FIG. 6 shows a perspective view of an embodiment of the interbody spinal implant having a generally oval shape and being especially well adapted for use in a cervical spine surgical procedure;

FIG. 7 shows an exploded view of a generally oval-shaped implant with an integration plate;

FIG. 8 shows an exploded view of a lateral lumbar implant with an integration plate;

FIG. 9 illustrates examples of types of process steps that can be used to form macro, micro, or nano processes;

FIG. 10 graphically represents the average amplitude, Ra;

FIG. 11 graphically represents the average peak-to-valley roughness, Rz;

FIG. 12 graphically represents the maximum peak-to-valley height, Rmax;

FIG. 13 graphically represents the total peak-to-valley of waviness profile; and

FIG. 14 graphically represents the mean spacing, Sm.

DETAILED DESCRIPTION OF THE INVENTION

The present invention provides for interbody spinal implants and processes of producing the implants. According to one embodiment of the present invention, the implant includes an integration surface having a roughened surface topography for at least a portion of the top surface, the bottom surface, or both surfaces. The integration surface comprises at least (a) a plurality of grains; (b) intergranular boundaries between the plurality of grains; and (c) unsatisfied chemical bonds. According to another embodiment of the present invention, the integration surface may be produced by texturing a surface by a chemical process, a mechanical processes, or both types of processes to provide the plurality of grains and intergranular boundaries and chemically etching the surface to provide unsatisfied chemical bonds. In addition, the plurality of grains and the intergranular boundaries may etch at different or non-uniform etch rates (e.g., the intergranular boundaries may etch at a faster rate than the grain bodies).

The invention may be further understood with reference to five different areas:

(1) intergranular chemistry; (2) boundary chemistry; (3) unsatisfied bonds; (4) chemistry of the subsurface; and (5) preservation of the surface chemistry. Although these topics are discussed in turn, each are not mutually exclusive and may fundamentally interrelate with one another.

(1) Intergranular Chemistry

When titanium (or a similar metal or alloy) is exposed to the atmosphere or oxygen (e.g., after cutting, lathing, milling, sawing, or the like) an oxide layer may form (e.g., titanium oxide, $TiO_x$, where x is a number in the range from 1.0 to 2.0). A surface oxide coating may form with a thickness of up to about 20 to 50 $\mu$m, for example. The characteristic composition and structure of the oxide layer may differ depending on the technique or techniques used to prepare the surface of the metal. In addition, the exact composition of the titanium oxide, the morphology, and the content or concentrations of other elements and impurities, for example, may be varied and controlled to provide the desired surface structure. In one embodiment, the chemical etching produces one or more oxide layers having a thicknesses in a range of 20 to 500 nanometers.

A solid metal, metal alloy, metal oxide, or metal hydroxide may be comprised of a plurality of grains (e.g., grain bodies). The plurality of grains may comprise individual grains of titanium, aluminum, or like metal or an alloy of the metal (e.g., titanium, aluminum, and vanadium). The plurality of grains may also include an oxide or hydroxide of the metal or alloy.

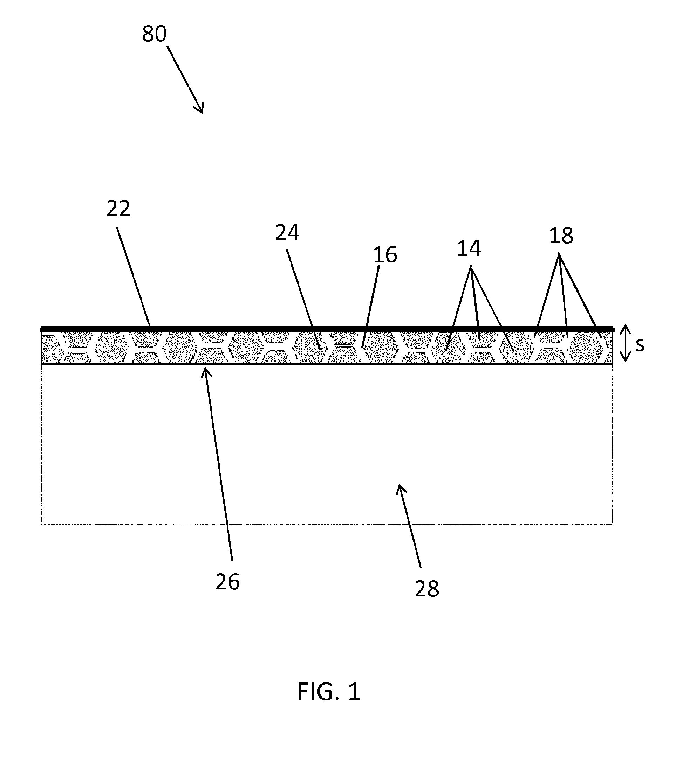

Referring now to the drawing, in which like reference numbers refer to like elements throughout the various figures that comprise the drawing, the microstructure of metals and alloys (and their oxides and hydroxides) may be made up of grains 14, separated by grain or intergranular boundaries 18. As graphically depicted in FIG. 1, the plurality of grains 14 may be composed of a grain body, which is bordered by a grain edge 16. The plurality of grains 14 may be structured, patterned, and oriented as will be recognized by one of ordinary skill in the art. The plurality of grains 14 may be arranged in a given relationship, such as a crystalline structure, or may be amorphous in nature. For example, a crystalline or polycrystalline structure defines a solid composed of atoms, ions, or molecules arranged in a pattern which is repetitive in three dimensions. The solid exhibits distinct x-ray diffraction intensity peaks characteristic of the crystal structure.

In the case of titanium, the titanium may include alpha and/or beta phases. The alpha phase of titanium is titanium in its pure state (e.g., pure titanium). The alpha phase may include an alpha stabilizer, which is an alloying element, such as gallium, germanium, carbon, oxygen, nitrogen, or combinations, that favors the alpha crystal structure. The beta phase of titanium is titanium that has been alloyed with beta stabilizers, such as molybdenum, silicon, vanadium, chromium, copper, or a combination, for example. An alpha-beta alloy results from mixing alpha and beta phases of titanium, for example, with alpha and beta stabilizers. There are seven types of titanium oxide, $TiO_x$, which may be formed on titanium materials (e.g., on the surface exposed to oxygen). They include (1) amorphous oxide, (2) cubic TiO ($a_o$ = 4.24 Å), (3) hexagonal $Ti_2O_3$ ($a_o$ = 5.371 Å, $\alpha$ = 56°48'), (4) tetragonal $TiO_2$ (anatase) ($a_o$ = 3.781 Å, $c_o$ = 9.50 Å), (5) tetragonal $TiO_2$ (rutile) ($a_o$ = 4.58 Å, $c_o$ = 2.98 Å), (6) orthorhombic $TiO_2$ (brookite) ($a_o$ = 9.171 Å, $b_o$ = 5.43 Å, $c_o$ = 5.131 Å), and (7) non-stoichiometric oxide. Although titanium is exemplified in this document, similar structures would be known for other metals, such as aluminum. There may also be other structures for titanium oxide alloys (e.g., containing aluminum, vanadium, and the like) or hydroxides.

The grains 14 may be of any suitable size, shape, and orientation needed to achieve the desired surface roughness and osteointegration. For example, the plurality of grains 14 may have an average diameter ranging up to about 20 $\mu$m, preferably from about 1-20 $\mu$m, more preferably from about 1-10 $\mu$m, or most preferably from about 1-5 $\mu$m. The grain size (e.g., an average diameter of a given sample) may be measured by techniques known in the art, for example, measurements by microscopic techniques, such as by a calibrated optical microscope, a scanning electron microscope, or other microscopic techniques. In an exemplary embodiment, the grains 14 comprise hexagonal crystals.

The oxides and hydroxides of titanium or other metal or alloy are located on the surface 22 and subsurface 24 of a bulk material 28 as a result of etch and other manufacturing processes described in this document. Once implanted, the surface 22 and the subsurface 24 (and perhaps the near subsurface 26) provide for a metal-to-organic interface, which occurs first, and then a cell-to-organic layer. The organic layer may help to mediate the actions of the bone forming cells. The roughening and texturing processes are selectively applied so as to optimize the biologic function of the surface 22 and the subsurface 24 and to preserve the formation of the other remaining surfaces.

(2) Boundary Chemistry

Without wishing to be bound by theory, it is believed that etching of the integration surface affects the surface 22 and the subsurface 24 at a microscopic level, specifically the intergranular boundaries 18 between the individual grains 14 (e.g., of the titanium and the alloy elements that compose the grains 14). In particular, the grain or intergranular boundaries 18 may etch at different or non-uniform rates from the body of the grain 14 providing for a structured surface that preferentially attracts the appropriate ambient organics. For example, the intergranular boundaries 18, the grains 14, and even the edges 16 of the grains 14 may etch at different rates with respect to one another. The organic materials, mostly proteins, align to the surface 22 and the subsurface 24 rapidly and in an appropriate orientation to enhance the attachment and differentiation of osteoblasts. (From the Greek words for "bone" and "germ" or embryonic, osteoblasts are mono-nucleate cells that are responsible for bone formation; in essence, osteoblasts are specialized fibroblasts that, in addition to fibroblastic products, express bone sialoprotein and osteocalcin.) Osteoblasts then attach to the organically coated and textured surface which allows for enhanced production of cytokines and growth factors that stimulate bone formation, such as bone morphogenic protein (BMP), vascular endothelial growth factor (VEGF), and other biological materials ambient in the joint space during initial healing.

Non-uniform etch rates may allow for differentiation of surface composition enhancing activities. Favorable cell responses in combination with surface topography stimulate successive biological processes required for bone formation. It is believed that points for chemical reaction which is followed by cellular attachment may be greater at the boundary 18 between the grains 14. For example, surface features resulting from etching are of appropriate shape and size (e.g., on the macro, micro, and nano scale) for connection by proteins and organics ambient in the disk space during initial healing. They serve as a precursor to stimulate osteblastic activation and function. In addition, smooth radius junctures without undercuts allow for attachment of proteins and then osteoblasts.

(3) Unsatisfied Bonds

As a result of production, open chemical bonds in contact with or proximate to vertebral endplates 25 are available for attachment of cellular materials during the healing process. After proteins act on the surface, specifically due to the post-production condition of the surface 22 and the subsurface 24, osteoblasts can form stable attachments with the surface 22, the subsurface 24, the near subsurface 26, or some combination of all three which allow for more rapid proliferation and differentiation of the osteoblasts to form bone. Surface energies, charges, and condition of molecules exposed after processing are designed to enhance functions of proteins and bone-forming cells.

A dangling bond may be an unsatisfied valence on an immobilized atom. In order to gain enough electrons to fill their valence shells (e.g., octet rule), many atoms will form covalent bonds with other atoms. In the simplest case, that of a single bond, two atoms each contribute one unpaired electron, and the resulting pair of electrons is shared between both atoms. Atoms which possess too few bonding partners to satisfy their valences and which possess unpaired electrons are termed free radicals; so, often, are molecules containing such atoms. When a free radical exists in an immobilized environment, for example, a solid, it is referred to as an "immobilized free radical" or a "dangling bond".

Free and immobilized radicals display very different chemical characteristics from atoms and molecules containing only complete bonds. Generally, they are extremely reactive. Immobilized free radicals, like their mobile counterparts, are highly unstable, but gain some kinetic stability because of limited mobility and steric hindrance. While free radicals are usually short lived, immobilized free radicals often exhibit a longer lifetime because of this reduction in reactivity. Some allotropes of metals may display a high concentration of dangling bonds. Hydrogen introduced to the metal during the synthesis process may replace dangling bonds.

The integration surface preferably includes a plurality of unsatisfied chemical bonds on the surface 22, the subsurface 24, the near subsurface 26, or some combination of the three. These unsatisfied chemical bonds may be in the form of hydroxylated reactive groups (a hydroxyl group -OH), for example. In addition, the integration surface and the unsatisfied chemical bonds may be hydrophilic in nature (e.g., charge-polarized and capable of hydrogen bonding). Alternatively, the integration surface and the unsatisfied chemical bonds may be hydrophobic in nature.

The unsatisfied chemical bonds may be provided in a hydrated environment to allow for more appropriate attachment of the organic materials to the integration surface. Reactive groups in a hydrated environment allow for more appropriate attachment of organics to which cell receptors more readily attach. In addition or in the alternative, the unsatisfied chemical bonds may be provided in a carbonated environment. For example, the integration surface may be provided in a hydrated and/or carbonated environment to allow for more appropriate attachment of the organic materials to the integration surface. Protein cells mature on the integration surface faster and signal osteoblastic activity. Polymeric materials, such as polyetheretherketone (PEEK), do not possess the molecular structure that can be modified, for example, with the macro, micro, nano processes, and transmit different signals to the biological materials in the joint space during healing due to the different nature of the organic layers interacting with the surface and successively with the osteoblast cells.

(4) Chemistry of Subsurface

The integration surface may include the outermost surface 22 (e.g., the surface in direct contact with the vertebral endplates 25), the subsurface 24 (e.g., an area below the outermost surface 22), and the near subsurface 26. The subsurface 24 may be non-uniform (e.g., in structure, orientation, thickness) and certain portions of the subsurface 24 may be directly or indirectly exposed to the vertebral endplate 25. For example, the subsurface 24 may be composed of the plurality of grains 14 having unsatisfied bonds, where the unsatisfied bonds are able to contact the vertebral endplate 25. In addition, the subsurface 24 may exist at a given distance or depth S into the remaining bulk material 28, which may or may not comprise grains 14. For example, the subsurface 24 may penetrate a distance S up to about 20 $\mu$m, preferably about 1-10 $\mu$m, and more preferably from about 1-5 $\mu$m. In one embodiment, the subsurface 24 exists at a distance or depth S of about the diameter of a single grain 14.

Exposed grains 14 and areas of the intergranular boundary 18 may differ due to the acid etching process, which may help to make organic bonding faster. Biologic materials may be able to penetrate even to the near subsurface 26 (e.g., up to about 20 $\mu$m). These biologic materials, ambient during healing, may be stimulated by available bonds, molecular geometry, and the chemistry of the grain 14 and boundaries 18 as a result of the macro, micro, and nano processing. The osteoblast cells have on their outer surface receptors that are integrins that bind to collagen or collagen-like proteins. (Integrins are trans-membrane receptors that mediate the attachment between a cell and the tissues that surround it.) The osteoblasts produce a collagen and mineralized matrix. The absorbed organic layer acts in a similar fashion to a collagen matrix which is a preferential structure for the attachment and differentiation of osteoblasts. Mixed organic attachment at boundaries 18 or grain faces (e.g., grain edge 16), orientation of grains, and as a result, spacing at atoms, cleavage planes, patterns and orientation of planes, allow or enhance organic attachment and subsequent osteoblast cell attachment. Grain sizes (e.g., grain size and boundary distances of about 1-20 $\mu$m) influence the shape and topography of the organics layer. A series of etches create an ideal combination of structure, chemistry, and surface energy. This surface organic layer will attach in proper orientation facilitating the attachment of osteoblasts and then stimulate osteoblasts to mature and produce bone at higher rates than on other surfaces. The sequence of events and healing process rates may be improved by surface charges, crystal structure spacing, topography, and chemical elements present on the integration surface. The etching steps used in the macro, micro, and nano processes create this ideal combination of structures.

(5) Preservation of the Surface Chemistry

The implant 1, 101, 101a, 201, and 301 may be subsequently cleaned and packaged to preserve the surface 22 (and the subsurface 24), the grains 14, the grain boundaries 18, and the unsatisfied chemical bonds. Part of the production process includes cleaning the implants 1, 101, 101a, 201, and 301 and, in particular, the integration surfaces, which helps to preserve the composition of the surface 22 and the subsurface 24. The implants 1, 101, 101a, 201, and 301 may be cleaned, for example, by washing in an aqueous environment under agitation and heated with or without a detergent. Preferably, the implants 1, 101, 101a, 201, and 301 are washed in an environment without pyrogens, organics, or inorganics. Following washing, the part may be dried, for example with hot air, heating in a dry oven, or both. Preferably, the surfaces are not exposed to any oils, greases, lubricants, or the like, which could affect the surface chemistry. After chemically etching the surface, the surface 22 may also be aseptically cleaned using plasma or other energy-based systems to refine the one or more oxide layers to provide a sterile condition and to preserve properties of the integration surface 22. After cleaning, the surface 22 is preferably aseptic in that the surface 22 is free from disease-causing contaminants, such as bacteria, viruses, fungi, and parasites and/or sterile meaning the surface 22 is free of all biological contaminants.

After cleaning, the implant 1, 101, 101a, 201, and 301 may be packaged to maintain the surface chemistry. Packaging can help to preserve the condition of the implant 1, 101, 101a, 201, and 301 during transportation and storage. For example, the packaging may include components that connect the implant 1, 101, 101a, 201, and 301 to the package to secure the implant 1, 101, 101a, 201, and 301 in position. In addition, the packaging may be designed to minimize contact of the implant 1, 101, 101a, 201, and 301 with the packaging materials. The packaging may also serve to contain liquids, gases, or an absence of certain liquids or gases within the container. In particular, atmospheres within the container may have a negative effect on the surface 22 and the subsurface 24. In an exemplary embodiment, the packaging may include preserving the interbody spinal implant 1, 101, 101a, 201, and 301 in an anaerobic environment (e.g., without or minimizing oxygen). Preferably, the implant 1, 101, 101a, 201, and 301 is packaged in a way to minimize oxidation of any or all surfaces.

Roughened Surface Topography

At least a portion of the top surface 10, 110, 110a, 210, and 310, the bottom surface 20, 120, 120a, 220, and 320 of the implant 1, 101, 101a, 201, and 301 (or the top surface 81, 381 of the integration plate 82, 382 when present), or both surfaces may each have a roughened surface topography 80, 180, 180a, 280, 380, without sharp teeth that risk damage to bone structures, adapted to grip bone through friction generated when the implant 1, 101, 101a, 201, and 301 is placed between two adjacent vertebral endplates 25, inhibit migration of the implant 1, 101, 101a, 201, and 301, and optionally promote biological and chemical fusion (e.g., a biostimulating effect).

The ability to achieve spinal fusion is directly related to the available vascular contact area over which fusion is desired, the quality and quantity of the fusion mass, and the stability of the interbody spinal implant 1, 101, 101a, 201, and 301. The implants 1, 101, 101a, 201, and 301 allow for improved seating over the apophyseal rim of the vertebral endplates 25 and better utilize this vital surface area over which fusion may occur and may better bear the considerable biomechanical loads presented through the spinal column with minimal interference with other anatomical or neurological spinal structures. The implants 1, 101, 101a, 201, and 301 may allow for improved visualization of implant seating and fusion assessment. The roughened surface topography 80, 180, 180a, 280, 380 helps to facilitate osteointegration (e.g., formation of a direct structural and functional interface between the artificial implant and living bone or soft tissue) with the surrounding living bone.

It is generally believed that the surface of an implant 1, 101, 101 a, 201, and 301 determines its ultimate ability to integrate into the surrounding living bone. Without being limited by theory, it is hypothesized that the cumulative effects of at least implant composition, implant surface energy, and implant surface roughness play a major role in the biological response to, and osteointegration of, an implant device. Thus, implant fixation may depend, at least in part, on the stimulation and proliferation of bone modeling and forming cells, such as osteoclasts and osteoblasts and like-functioning cells upon the implant surface. Still further, it appears that these cells attach more readily to relatively rough surfaces rather than smooth surfaces. In this manner, a surface may be bioactive due to its ability to stimulate cellular attachment and osteointegration. The roughened surface topography 80, 180, 180a, 280, 380 described in this document may better promote the osteointegration of certain embodiments of the present invention. The roughened surface topography 80, 180, 180a, 280, 380 may also better grip the surfaces of the vertebral endplate 25 and inhibit implant migration upon placement and seating.

The implant 1, 101, 101a, 201, and 301 may include the roughened surface topography 80, 180, 180a, 280, 380 on at least a portion of one or more integration surfaces. As used in this document, the integration surface is the surface at least partially in contact with the bone structure or vertebral endplates 25 (e.g., the top surface 10, 110, 110a, 210, and 310 or bottom surface 20, 120, 120a, 220, and 320 of the implant 1,101, 101a, 201, and 301 or the top surface 81, 381 of the integration plate 82, 382 when present).

The roughened surface topography 80, 180, 180a, 280, 380 preferably contains predefined surface features that (a) engage the vertebral endplates 25 with a friction fit and, following an endplate preserving surgical technique, (b) attain initial stabilization, and (c) benefit fusion. The composition of the vertebral endplate 25 is a thin layer of notch-sensitive bone that is easily damaged by features (such as teeth) that protrude sharply from the surface of traditional implants. Avoiding such teeth and the attendant risk of damage, the roughened surface topography 80, 180, 180a, 280, 380 does not have teeth or other sharp, potentially damaging structures; rather, the roughened surface topography 80, 180, 180a, 280, 380 may have a pattern of repeating features of predetermined sizes, smooth shapes, and orientations. By "predetermined" is meant determined beforehand, so that the predetermined characteristic of the surface must be determined, i.e., chosen or at least known, before use of the implant 1, 101, 101a, 201, and 301.

The roughened surface topography 80, 180, 180a, 280, 380 may be comprised of macro features, micro features, and nano features. For example, the roughened surface topography 80, 180, 180a, 280, 380 may be obtained by combining separate macro processing, micro processing, and nano processing steps. The term "macro" typically means relatively large; for example, in the present application, dimensions measured in millimeters (mm). The term "micro" typically means one millionth ($10^{-6}$); for example, in the present application, dimensions measured in microns ($\mu$m) which correspond to $10^{-6}$ meters. The term "nano" typically means one billionth ($10^{-9}$); for example, in the present application, dimensions measured in nanometers (nm) which correspond to $10^{-9}$ meters.

The shapes of the frictional surface protrusions of the roughened surface topography 80, 180, 180a, 280, 380 may be formed using processes and methods commonly applied to remove metal during fabrication of implantable devices such as chemical, electrical, electrochemical, plasma, or laser etching; cutting and removal processes; casting; forging; machining; drilling; grinding; shot peening; abrasive media blasting (such as sand or grit blasting); and combinations of these subtractive processes. Additive processes such as welding, thermal, coatings, sputtering, and optical melt additive processes are also suitable. The resulting surfaces either can be random in the shape and location of the features or can have repeating patterns. This flexibility allows for the design and production of surfaces that resist motion induced by loading in specific directions that are beneficial to the installation process and resist the opposing forces that can be the result of biologic or patient activities such as standing, bending, or turning or as a result of other activities.

The shapes of the surface features when overlapping increase the surface contact area but do not result in undercuts that generate a cutting or aggressively abrasive action on the contacting bone surfaces.

These designed surfaces are composed of various sizes of features that, at the microscopic level, interact with the tissues and stimulate their natural remodeling and growth. At a larger scale these features perform the function of generating non-stressful friction that, when combined with a surgical technique that retains the most rigid cortical bone structures in the disc space, allow for a friction fit that does not abrade, chip, perforate, or compromise the critical endplate structures. The features may be divided into three size scales: nano, micro, and macro. The overlapping of the three feature sizes can be achieved using manufacturing processes that are completed sequentially and, therefore, do not remove or degrade the previous method.

The first step in the process may be mechanical (e.g., machining though conventional processes) or chemical bulk removal, for example, to generate macro features. The macro features may be of any suitable shape, for example, roughly spherical in shape, without undercuts or protruding sharp edges. Other shapes are possible, such as ovals, polygons

(including rectangles), and the like. These features may be at least partially overlapped with the next scale (micro) of features using either chemical or mechanical methods (e.g., $AlO_2$ blasting) in predetermined patterns which also do not result in undercuts or protruding sharp edges. The third and final process step is completed through more mild (less aggressive) etching (e.g., HCl acid etching) that, when completed, generates surface features in both the micro and nano scales over both of the features generated by the two previous steps. The nano layer dictates the final chemistry of the implant material.

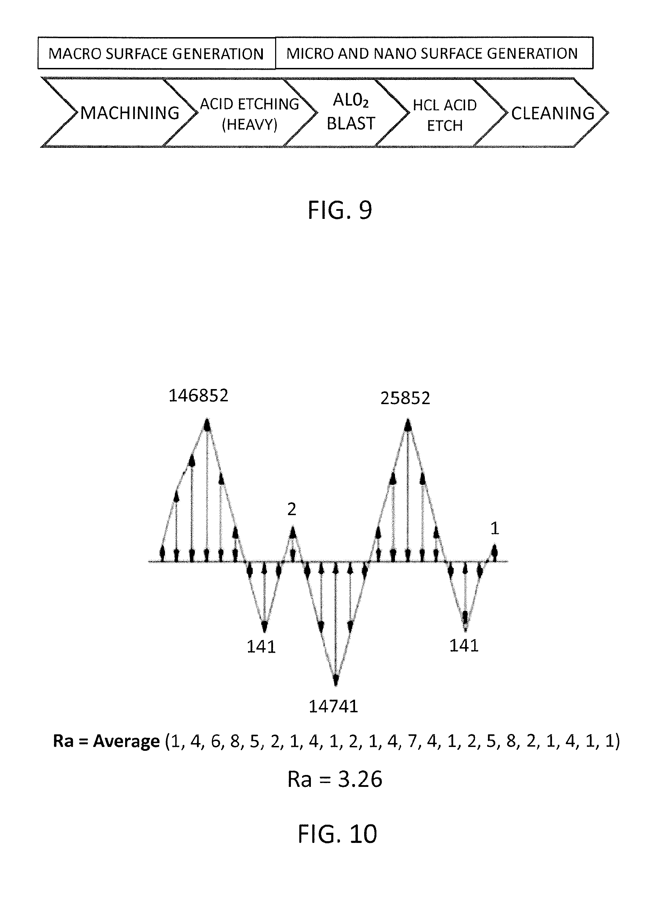

FIG. 9 illustrates one set of process steps that can be used to form the roughened surface topography 80, 180, 180a, 280, 380 according to an embodiment of the present invention. First, the part is machined, for example, from a bar stock comprising titanium, and a rough clean may be provided to remove any contaminants from machining. Second, the part may undergo a heavy acid etching (e.g., masked etching). Next, the part may undergo an abrasive blast, for example, using an alumina abrasive. The part may also undergo another acid etch, for example, with a solution comprising hydrochloric acid. Finally, the part may undergo a cleaning (e.g., with water and optionally a detergent). As illustrated, there may be some overlap in the processes that can be applied to form each of the three types of features (macro, micro, and nano). For example, acid etching can be used to form the macro features, then the same or a different acid etching process can be used to form the micro features.

(a) Macro Features

The macro features of the roughened surface topography 80, 180, 180a, 280, 380 are relatively large features (e.g., on the order of millimeters). The macro features may be formed from subtractive techniques (e.g., mechanical or chemical bulk removal, for example) or additive techniques (e.g., deposition). Preferably, the macro features are formed by subtractive techniques, which remove portions of the surface (e.g., from the base material that was used to form the implant 1, 101, 101a, 201, and 301). Suitable subtractive techniques may include, for example, machining (e.g., machine tools, such as saws, lathes, milling machines, and drill presses, are used with a sharp cutting tool to physically remove material to achieve a desired geometry) or masked etching (e.g., portions of the surface are protected by a "masking" material which resists etching and an etching substance is applied to unmasked portions). The patterns may be organized in regular repeating patterns and optionally overlap each other. In a preferred embodiment, the macro features may be formed in three, sequential steps.

The macro features may be produced by a heavy masked etching process, for example. Before etching, the surface may be cleaned and optionally blasted with an abrasive (e.g., alumina) in the areas to be chemically textured. Certain areas may be masked in a pattern using an etch resist and cured. The surface may then be chemically milled, for example, using a composition comprising hydrofluoric acid. The maskant and chemical milling may be repeated any number of times necessary to produce the desired pattern and etching depth. After the final etching process, the maskant may be removed and the part may be cleaned. The surface may also be passivated, for example, using an aqueous solution comprising nitric acid. The part may be cleaned and rinsed with water.

The macro features may be formed, for example, using three cut patterns. Specifically, a first cut pattern of the macro features may be formed in a surface (e.g., the top surface 10, 110, 110a, 210, and 310). The "cut 1" features of the first cut pattern may cover about 20% of the total area of the surface, for example, leaving about 80% of the original surface remaining. The range of these percentages may be about ± 20%, preferably ± 10%, and more preferably about ± 5%. The "cut 1" features of the first cut pattern do not have any undercuts. In one embodiment, these "cut 1" features have the smallest diameter and greatest depth of the macro features that are formed during the sequential steps.

A second cut pattern of the macro features may be formed in the surface. Together, the "cut 1" features of the first cut pattern and the "cut 2" features of the second cut pattern may cover about 85% of the total area of the surface, for example, leaving about 15% of the original surface remaining. The range of these percentages may be about ± 10% and preferably ± 5%. In an embodiment of the invention, these "cut 2" features have both a diameter and a depth between those of the "cut 1" and "cut 3" features of the macro features that are formed during the first and third steps of the process of forming the macro features of the roughened surface topography 80, 180, 180a, 280, 380.

A third cut pattern of the macro features may be formed in the surface. Together, the "cut 1" features of the first cut pattern, the "cut 2" features of the second cut pattern, and the "cut 3" features of the third cut pattern may cover about 95% of the total area of the surface, for example, leaving about 5% of the original surface remaining. The range of these percentages may be about ± 1%. In an embodiment of the invention, these "cut 3" features may have the largest diameter and least depth of the macro features that are formed during the sequential process steps.

(b) Micro Features

After the macro features are formed, additional process steps may be sequentially applied, in turn, to form the micro surface features (e.g., on the order of micrometers) of the roughened surface topography 80, 180, 180a, 280, and 380. The micro features may also be formed from subtractive techniques (e.g., mechanical or chemical bulk removal, for example) or additive techniques (e.g., deposition). Preferably, the micro features are also formed by subtractive techniques.

In an exemplary embodiment, the micro features are removed by masked or unmasked etching, such as acid etching. For example, portions of the surface, including portions of the surface exposed by the macro step(s) described above, may be exposed to abrasive blasting, chemical etching, or both. In an exemplary embodiment, the micro process includes an acid etching, with a strong acid, such as hydrochloric acid (HCl), hydroiodic acid (HI), hydrobromic acid (HBr), hydrofluoric (HF), perchloric acid ($HClO_4$), nitric acid ($HNO_3$), sulfuric acid ($H_2SO_4$), and the like. Preferably, the acid etching uses an aqueous solution comprising hydrochloric acid. The etching process may be repeated a number of times as necessitated by the amount and nature of the irregularities required for any particular application. Control of the strength of the etchant material, the temperature at which the etching process takes place, and the time allotted for the etching process allows fine control over the resulting surface produced by the process. The number of repetitions of the etching process can also be used to control the surface features. For example, the roughened surface topography 80, 180, 180a, 280, and 380 may be obtained via the repetitive masking and chemical or electrochemical milling processes described in U.S. Patents No. 5,258,098; No. 5,507,815; No. 5,922,029; and No. 6,193,762, the contents of which are incorporated by reference into this document, in their entirety, and for all purposes.

By way of example, an etchant mixture of at least one of nitric acid and hydrofluoric acid may be repeatedly applied to a titanium surface to produce an average etch depth of about 0.53 mm. In another example, chemical modification of titanium can be achieved using at least one of hydrofluoric acid, hydrochloric acid, and sulfuric acid. In a dual acid etching process, for example, the first exposure is to hydrofluoric acid and the second is to a hydrochloric acid and sulfuric acid mixture. Chemical acid etching alone may enhance osteointegration without adding particulate matter (e.g., hydroxyapatite) or embedding surface contaminants (e.g., grit particles).

In one embodiment, the micro features are created by abrasive or grit blasting, for example, by applying a stream of abrasive material (such as alumina, sand, and the like) to the surface. In an exemplary embodiment, the micro features are created, at least partially, with an aqueous hydrochloric acid etching step and at least partially with an $AlO_2$ blasting step. Patterns may be organized in regular repeating patterns and optionally overlap each other. After the micro features are formed, it is possible that less than about 3% of the original surface remains. The range of that percentage may be about ±1%.

(c) Nano Features

After the macro features and micro features are formed, additional process steps may be sequentially applied, in turn, to form the nano surface features (e.g., on the order of nanometers) of the roughened surface topography 80, 180, 180a, 280, and 380. The nano features may also be formed from subtractive techniques (e.g., mechanical or chemical bulk removal, for example) or additive techniques (e.g., deposition). Preferably, the nano features are also formed by subtractive techniques.

In an exemplary embodiment, the nano features are removed by masked or unmasked etching. For example, portions of the surface, including portions of the surface exposed by the macro and micro steps described above, may be exposed to a chemical etching. In an exemplary embodiment, the nano process also includes an acid etching, with a strong or weak acid, such as hydrochloric acid (HCl), hydroiodic acid (HI), hydrobromic acid (HBr), hydrofluoric (HF), perchloric acid ($HClO_4$), nitric acid ($HNO_3$), sulfuric acid ($H_2SO_4$), and the like. The acid etching process for the nano step is preferably less aggressive than the acid etching process in the macro or micro steps. In other words, a less acidic, mild, or more diluted acid may be selected. In an exemplary embodiment, the nano features are created, at least partially, with an aqueous hydrochloric acid etching step.

As an example, the nano features (or micro features) may be formed by preparing an acid solution comprising hydrochloric acid, water, and titanium; applying the acid solution to the surface; removing the acid solution by rinsing with water; and heating and subsequently cooling the surface.

The acid solution may be prepared using any suitable techniques known in the art. For example, the acid solution may be prepared by combining hydrochloric acid and water, simultaneously or sequentially. The aqueous hydrochloric acid solution may optionally be heated, for example, to a temperature of about 150-250°F (66-121°C), preferably about 200-210°F (93-99°C), and most preferably about 205°F (96°C). The titanium may be seeded (e.g., added) in the aqueous hydrochloric acid solution or may already be present from titanium previously removed from at least one surface of the implant 1, 101, 101a, 201, and 301, for example, in a continuous manufacturing process. The solution may optionally be cooled. The acid solution may comprise a concentration of 20-40% hydrochloric acid, preferably about 25-31 % hydrochloric acid, and more preferably about 28% hydrochloric acid, based on the weight percent of the solution.

The acid solution may be applied to the surface using any suitable mechanism or techniques known in the art, for example, immersion, spraying, brushing, and the like. In an exemplary embodiment, the acid solution is applied by immersing the entire part in the solution. It is also contemplated that the surface may be immersed in the acid solution alone or in combination with the assembled implant 1, 101, 101a, 201, and 301. If desired, certain areas of the surface or the implant 1, 101, 101a, 201, and 301 may be masked in patterns or to protect certain portions of the implant 1, 101, 101a, 201, and 301. The acid solution may be heated when it is applied. For example, the solution may be heated to a temperature of about 150-250°F (66-121°C), preferably about 200-210°F (93-99°C), and most preferably about 205°F (96°C). The solution may also be applied for any suitable period of time. For example, the solution may be applied for a period of time of about 5-30 minutes, preferably about 15-25 minutes, and more preferably about 20 minutes.

After the acid solution is applied, the acid solution may be removed, for example, by rinsing with water (e.g., deionized water). The surface or entire implant 1, 101, 101a, 201, and 301 may be subsequently dried. The surface may be dried using any suitable mechanism or techniques known in the art, for example, by heating in an oven (e.g., a dry oven). The surface may be heated to a temperature of about 110-130°F (43-54°C), preferably about 120-125°F (49-52°C), and most preferably about 122.5°F (50°C). The surface may be heated for any suitable period of time, for example about 30-50 minutes, preferably about 35-45 minutes, and more preferably about 40 minutes. After heating, the surface may be cooled to room temperature, for example.

It is contemplated that the nano features may also be created by the abrasive or grit blasting, for example, described for the micro processing step. Patterns may be organized in regular repeating patterns and optionally overlap each other. The nano features may also be achieved by tumble finishing (e.g., tumbling) the part or the implant 1, 101, 101a, 201, and 301. Suitable equipment and techniques can be selected by one of ordinary skill in the art. For example, a barrel may be filled with the parts or implants 1, 101, 101a, 201, and 301 and the barrel is then rotated. The parts or implants 1, 101, 101a, 201, and 301 may be tumbled against themselves or with steel balls, shot, rounded-end pins, ballcones, or the like. The tumbling process may be wet (e.g., with a lubricant) or dry. After the nano features are formed, it is possible that less than about 1% of the original surface remains. For example, after the nano features are formed, the roughened surface topography 80, 180, 180a, 280, and 380 may cover substantially all of the top surface 10, 110, 110a, 210, and 310 and/or bottom surface 20, 120, 120a, 220, and 320 of the implant 1, 101, 101a, 201, and 301 in contact with the vertebral endplate 25 (except for the rounded edges connected to the lateral 30, 130, 130a, 230, 330 and posterior portions 50, 150, 150, 250, and 350.

Any or each of the steps, including the macro, micro, or nano processing steps, may be accompanied by a cleaning step. In addition, the part may be cleaned once the processing steps are complete. For example, the part may be washed in an aqueous environment under agitation and heat with or without a detergent. Following washing, the part may be dried, for example with hot air, heating in a dry oven, or both.

As should be readily apparent to a skilled artisan, the process steps described in this document can be adjusted to create a mixture of depths, diameters, feature sizes, and other geometries suitable for a particular implant application. The orientation of the pattern of features can also be adjusted. Such flexibility is desirable, especially because the ultimate pattern of the roughened surface topography 80, 180, 180a, 280, and 380 of the implant 1, 101, 101a, 201, and 301 should be oriented in opposition to the biologic forces on the implant 1, 101, 101a, 201, and 301 and to the insertion direction. In one particular embodiment, for example, the pattern of the roughened surface topography 80, 180, 180a, 280, and 380 may be modeled after an S-shaped tire tread.

Roughness Parameters

Several separate parameters can be used to characterize the roughness of an implant surface. Among those parameters are the average amplitude, Ra; the maximum peak-to-valley height, Rmax; and the mean spacing, Sm. Each of these three parameters, and others, are explained in detail below. Surface roughness may be measured using a laser profilometer or other standard instrumentation.

In addition to the parameters Ra, Rmax, and Sm mentioned above, at least two other parameters can be used to characterize the roughness of an implant surface. In summary, the five parameters are: (1) average amplitude, Ra;

(2) average peak-to-valley roughness, Rz; (3) maximum peak-to-valley height, Rmax; (4) total peak-to-valley of waviness profile, Wt; and (5) mean spacing, Sm. Each parameter is explained in detail as follows.

### 1. Average Amplitude Ra

In practice, "Ra" is the most commonly used roughness parameter. It is the arithmetic average height. Mathematically, Ra is computed as the average distance between each roughness profile point and the mean line. In FIG. 10, the average amplitude is the average length of the arrows.

In mathematical terms, this process can be represented as

$$Ra = \frac{1}{n} \sum_{i=1}^{n} |y_i|$$

### 2. Average Peak-to-Valley Roughness Rz

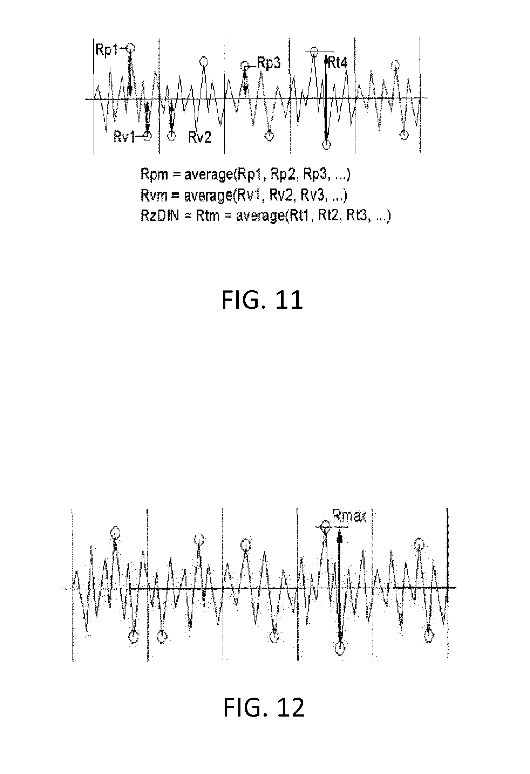

The average peak-to-valley roughness, Rz, is defined by the ISO and ASME 1995 and later. Rz is based on one peak and one valley per sampling length. The RzDIN value is based on the determination of the peak-to-valley distance in each sampling length. These individual peak-to-valley distances are averaged, resulting in the RzDIN value, as illustrated in FIG. 11.

### 3. Maximum Peak-to-Valley Height Rmax

The maximum peak-to-valley height, Rmax, is the maximum peak-to-valley distance in a single sampling length -- as illustrated in FIG. 12.

### 4. Total Peak-to-Valley of Waviness Profile Wt

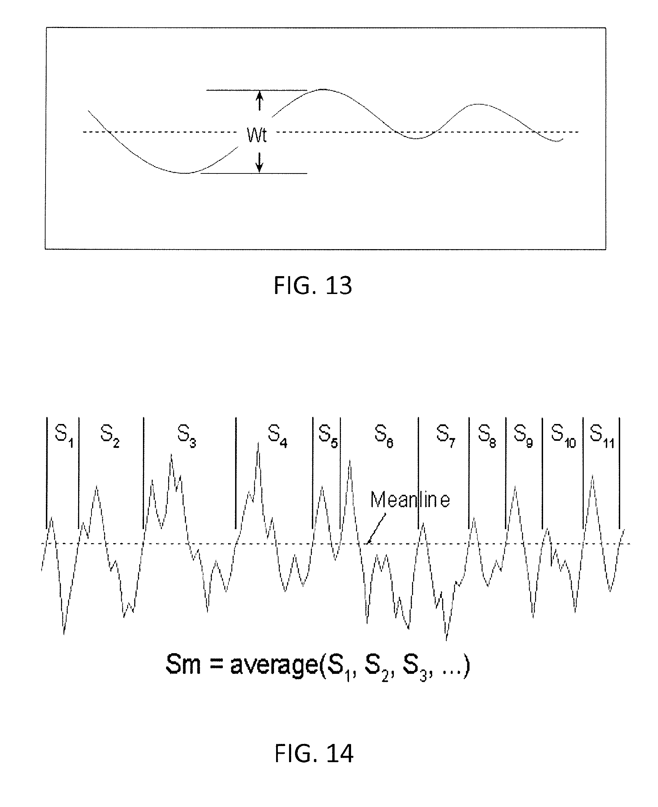

The total peak-to-valley of waviness profile (over the entire assessment length) is illustrated in FIG. 13.

### 5. Mean Spacing Sm

The mean spacing, Sm, is the average spacing between positive mean line crossings. The distance between each positive (upward) mean line crossing is determined and the average value is calculated, as illustrated in FIG. 14.

The parameters Sm, Rmax, and Ra can be used define the surface roughness following formation of each of the three types of features macro, micro, and nano. Such data are provided in Table 1 below.

TABLE 1: EXAMPLE DATA BY PROCESS STEP

Surface Feature Size and Roughness (Metric): Macro ($\mu$m)

|      | Size (Sm) | Depth (Rmax) | Roughness (Ra) |
|------|-----------|--------------|----------------|
| Max. | 2,000     | 500          | 200            |
| Min. | 400       | 40           | 20             |
| Avg. | 1,200     | 270          | 110            |

Surface Feature Size and Roughness (Metric): Micro ($\mu$m)

|      | Size (Sm) | Depth (Rmax) | Roughness (Ra) |
|------|-----------|--------------|----------------|
| Max. | 400       | 40           | 20             |
| Min. | 20        | 2            | 1              |

(continued)

|  | Size (Sm) | Depth (Rmax) | Roughness (Ra) |
|---|---|---|---|
| Avg. | 210 | 11 | 5.5 |

Surface Feature Size and Roughness (Metric): Nano ($\mu$m)

|  | Size (Sm) | Depth (Rmax) | Roughness (Ra) |
|---|---|---|---|
| Max. | 20 | 2 | 1 |
| Min. | 0.5 | 0.2 | 0.01 |
| Avg. | 10.25 | 1.1 | 0.505 |

From the data in Table 1, the following preferred ranges (all measurements in microns) can be derived for the macro features for each of the three parameters. The mean spacing, Sm, is between about 400-2,000, with a range of 750-1,750 preferred and a range of 1,000-1,500 most preferred. The maximum peak-to-valley height, Rmax, is between about 40-500, with a range of 150-400 preferred and a range of 250-300 most preferred. The average amplitude, Ra, is between about 20-200, with a range of 50-150 preferred and a range of 100-125 most preferred.

The following preferred ranges (all measurements in microns) can be derived for the micro features for each of the three parameters. The mean spacing, Sm, is between about 20-400, with a range of 100-300 preferred and a range of 200-250 most preferred. The maximum peak-to-valley height, Rmax, is between about 2-40, with a range of 2-20 preferred and a range of 9-13 most preferred. The average amplitude, Ra, is between about 1-20, with a range of 2-15 preferred and a range of 4-10 most preferred.

The following preferred ranges (all measurements in microns) can be derived for the nano features for each of the three parameters. The mean spacing, Sm, is between about 0.5-20, with a range of 1-15 preferred and a range of 5-12 most preferred. The maximum peak-to-valley height, Rmax, is between about 0.2-2, with a range of 0.2-1.8 preferred and a range of 0.3-1.3 most preferred. The average amplitude, Ra, is between about 0.01-1, with a range of 0.02-0.8 preferred and a range of 0.03-0.6 most preferred.

Implant Design

The spinal implant 1, 101, 101a, 201, and 301 includes the top surface 10, 110, 110a, 210, and 310, the bottom surface 20, 120, 120a, 220, and 320, opposing lateral sides 30, 130, 130a, 230, and 330, and opposing anterior 40, 140, 140a, 240, and 340 and posterior 50, 150, 150a, 250, and 350 portions. The implant 1, 101, 101a, 201, and 301 may be of any suitable shape. For example, the body of the implant 1, 101, 101a, 201, and 301 may have a generally oval shape, a generally rectangular shape, a generally curved shape, or any other shape described or exemplified in this specification.

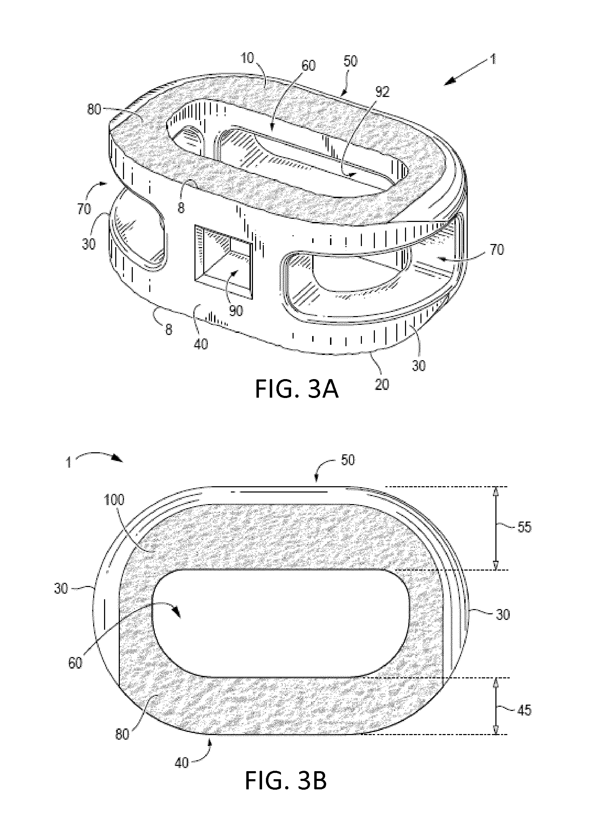

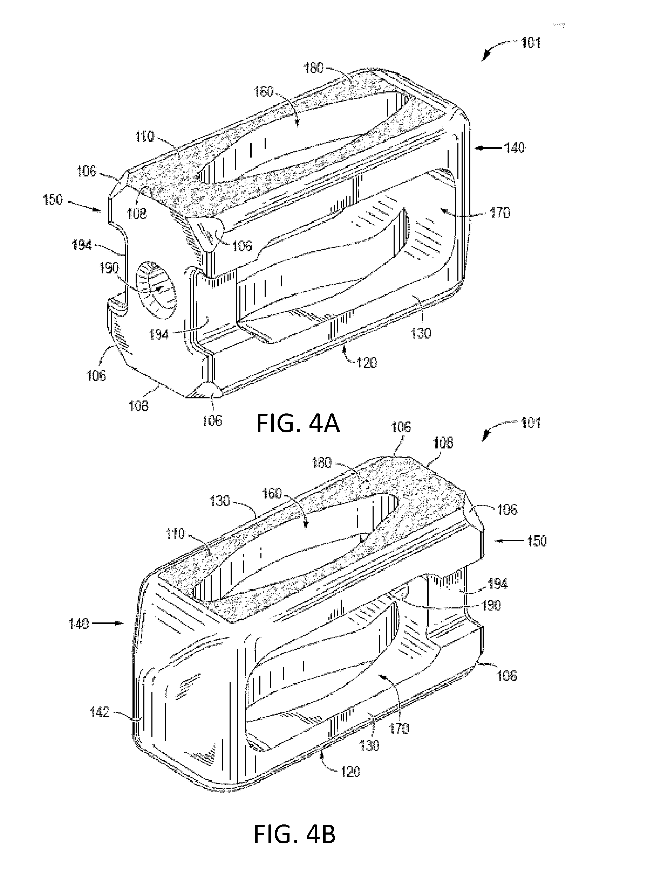

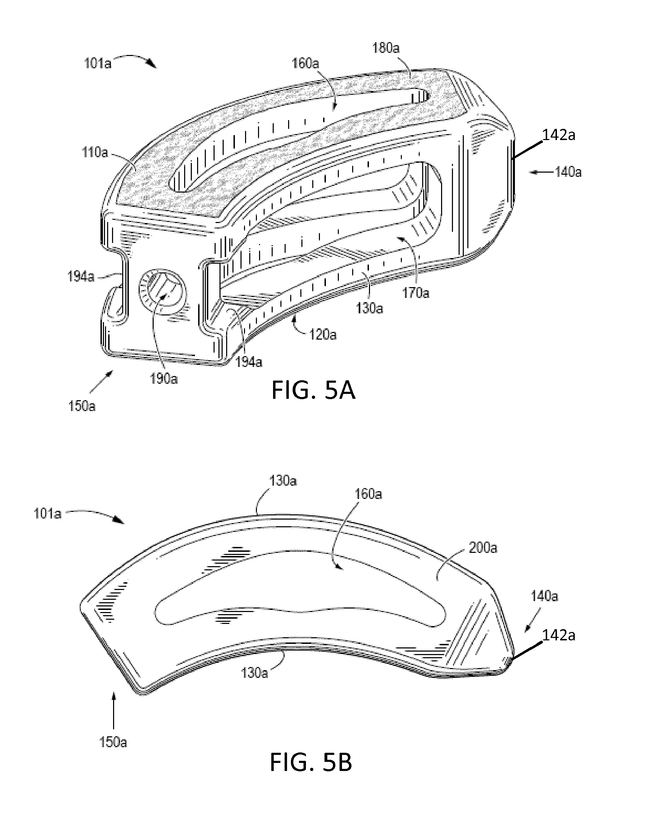

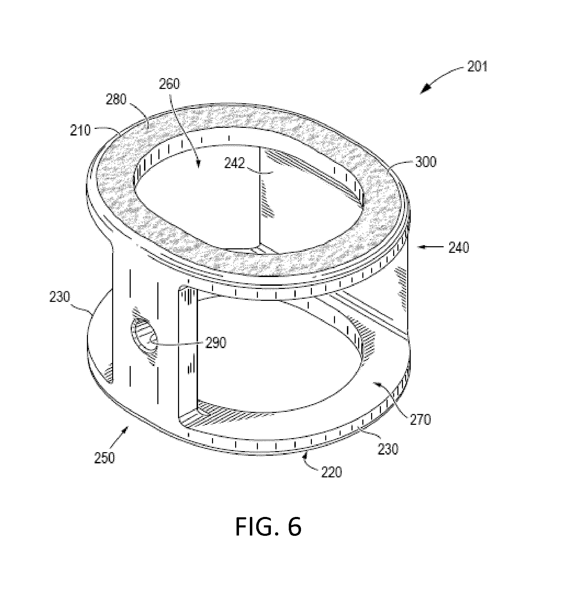

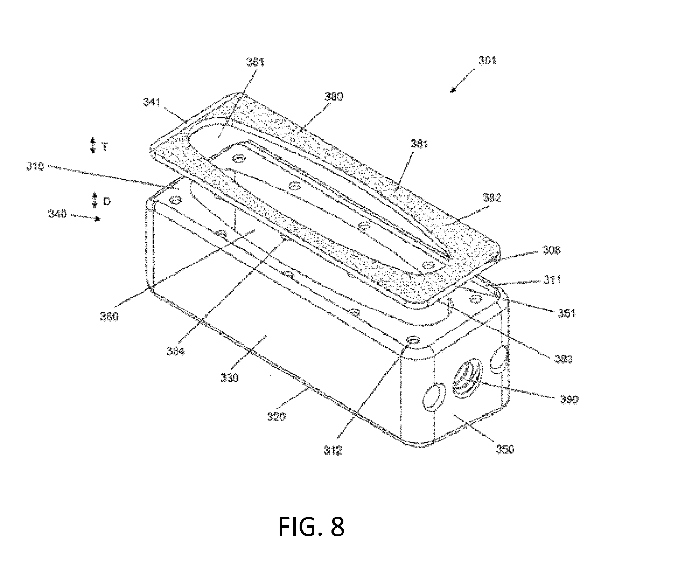

Certain embodiments of the interbody implant 1 have a generally oval-shaped transverse cross-sectional area (e.g., FIG. 3A), which may be suitable for Anterior Lumbar Interbody Fusion (ALIF). The implant 101 may have a generally rectangular transverse cross-sectional area (e.g., FIG. 4A) suitable for PLIF. The implant 101a may have a generally curved shape (e.g., FIG. 5A) suitable for TLIF fusion. The implant 201 may be generally circular in shape (e.g., FIG. 6) suitable for cervical fusion. The implant 301 may be generally rectangular in shape (e.g., FIG. 8) suitable for lateral lumbar insertion. The implant 1, 101, 101a, 201, and 301 may be shaped to reduce the risk of subsidence, and improve stability, by maximizing contact with the apophyseal rim of vertebral endplates 25. Embodiments may be provided in a variety of anatomical footprints and sizes.

The implant 1, 101, 101a, 201, and 301 may comprise one or more apertures (see, e.g., FIGS 3A, 3B, 4A, 4B, 5A, 5B, 6, 7, and 8). For example, the implant 1,101, 101a, 201, and 301 may comprise one or more apertures which extend through the body of the implant 1, 101, 101a, 201, and 301. The implant 1, 101, 101a, 201, and 301 may include one or more vertical apertures 60, 160, 160a, 260, and 360 extending through the main body of the implant 1, 101, 101a, 201, and 301, respectively. In an exemplary embodiment, the implant 1, 101, 101a, 201, and 301 includes a single vertical aperture 60, 160, 160a, 260, and 360 which (a) extends from the top surface 10, 110, 110a, 210, and 310 to the bottom surface 20, 120, 120a, 220, and 320, (b) has a size and shape predetermined to maximize the surface area of the top surface 10, 110, 110a, 210, and 310 and the bottom surface 20, 120, 120a, 220, and 320 available proximate the anterior 40, 140, 140a, 240, and 340 and posterior 50, 150, 150a, 250, and 350 portions while maximizing both radiographic visualization and access to the substantially hollow center, and optionally (c) defines a transverse rim 100, 200a, and 300. The interbody implant 1 having a generally oval-shaped transverse cross-sectional area (e.g., FIG. 3A) may include the single vertical aperture 60 having a substantially D-shaped cross-section.

The transverse rim 100 defined by the vertical aperture 60 may have a greater posterior portion thickness 55 than an anterior portion thickness 45 (see, e.g., FIGS. 3A and 3B). In at least one embodiment, the opposing lateral sides 30 and the anterior portion 40 have a rim thickness 45 of about 5 mm, while the posterior portion 50 has a rim thickness 55 of about 7 mm. Thus, the rim posterior portion thickness 55 may allow for better stress sharing between the implant 1 and the adjacent vertebral endplates 25 and helps to compensate for the weaker posterior vertebral endplate 25. In some aspects, the transverse rim 100 has a generally large surface area and contacts the vertebral endplate 25. The transverse rim 100 may act to better distribute contact stresses upon the implant 1, and hence minimize the risk of subsidence while maximizing contact with the apophyseal supportive bone. It is also possible for the transverse rim 100 to have a substantially constant thickness (e.g., for the anterior portion thickness 45 to be substantially the same as the posterior portion thickness 55) or for the posterior portion 50 to have a rim thickness 55 less than that of the opposing lateral sides 30 and the anterior portion 40.

FIG. 5A illustrates a perspective view of the implant 101a with a curved transverse rim 200a. The width of the transverse rim 200a is 9 mm in the regions adjacent the anterior 140a and posterior 150a portions. That width gradually increases to 11 mm, however, near the center of the transverse rim 200a. The additional real estate provided by the transverse rim 200a (relative to the transverse rim 100) allows the shape of the vertical aperture 160a to change, in cross section, from approximating a football to approximating a boomerang. Maintaining the thickness of the transverse rim 200a on either side of the vertical aperture 160a adjacent the center of the vertical aperture 160a at about 2 mm, the center of the vertical aperture 160a, which defines the maximum width of the vertical aperture 160a, is increased (from 5 mm for the implant 101) to about 7 mm. FIG. 6 illustrates a perspective view of the implant 201 where vertical aperture 260 further defines the transverse rim 300. In one example, the vertical aperture 60, 160, 160a, 260, and 360 may define the transverse rim 100, 200a, and 300 with a varying width or thickness, and having a maximum width at its center, between the opposing lateral sides 30, 130, 130a, 230, and 330, ranging between about 55% and 64% of the distance between the opposing lateral sides 30, 130, 130a, 230, and 330 and tapering inwardly from the center to each of its ends, one end proximate the anterior portion 40, 140, 140a, 240, and 340 and the other end proximate the posterior portion 50, 150, 150a, 250, and 350.

Certain embodiments of the interbody implant 1, 101, 101a, 201, and 301 are substantially hollow. Substantially hollow, as used in this document, means at least about 33% of the interior volume of the interbody spinal implant 1, 101, 101 a, 201, and 301 is vacant. The substantially hollow portion may be filled, for example, with cancellous autograft bone, allograft bone, demineralized bone matrix (DBM), porous synthetic bone graft substitute, bone morphogenic protein (BMP), or combinations of those materials.

The implant 1, 101, 101a, 201, and 301 may further comprise one or more transverse apertures 70, 170, 170a, and 270. The transverse aperture 70, 170, 170a, and 270 may extend the entire transverse length of the body of the implant 1, 101, 101a, 201, and 301. The transverse aperture 70, 170, 170a, and 270 may provide improved visibility of the implant 1, 101, 101a, 201, and 301 during surgical procedures to ensure proper implant placement and seating, and may also improve post-operative assessment of implant fusion. The transverse aperture 70, 170, 170a, and 270 may be broken into two, separate sections by an intermediate wall. Suitable shapes and dimensions for the transverse aperture 70, 170, 170a, and 270 may be selected by one of ordinary skill in the art. In particular, all edges of the transverse aperture 70, 170, 170a, and 270 may be rounded, smooth, or both. The intermediate wall may be made of the same material as the remainder of the body of the implant 1, 101, 101a, 201, and 301 (e.g., titanium), or it may be made of another material (e.g., plastic). The intermediate wall may offer one or more of several advantages, including reinforcement of the implant 1, 101, 101a, 201, and 301 and improved bone graft containment.

In the alternative, the implant 1, 101, 101a, 201, and 301 may comprise a solid body, for example, containing no apertures or openings extending through the implant 1, 101, 101a, 201, and 301 (e.g., in the vertical or transverse directions). The implants 1,101, 101a, 201, and 301 may contain openings (e.g., an opening 90), however, in one or more surfaces of the implant 1, 101, 101 a, 201, and 301, for example, for manipulation by tools and the like.

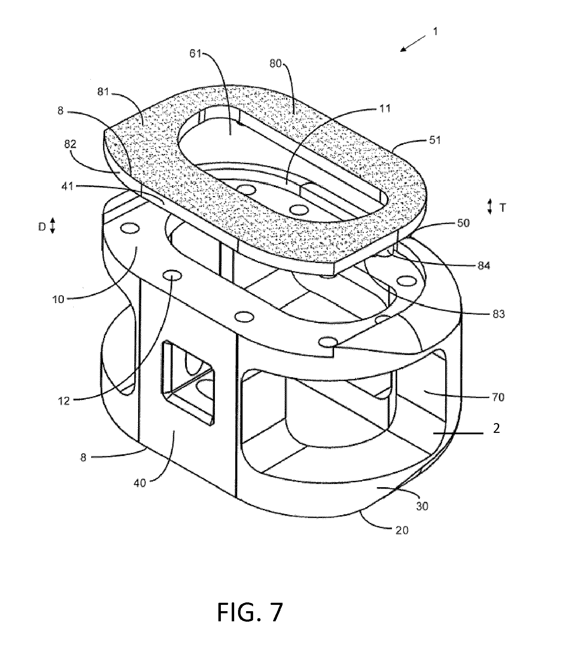

The implant 1, 101, 101a, 201, and 301 may be formed from a single material or may be formed as a composite made from more than one type of material. As depicted in FIGS. 7 and 8, a composite implant 1, 101, 101a, 201, and 301 may comprise one or two integration plates 82, 382, for example. The implant 1, 101, 101a, 201, and 301 may include a first integration plate 82, 382 affixed to and recessed or inset into the top surface 10, 310 of the body 2 and an optional second integration plate 82, 382 (not shown) affixed to and recessed or inset into the bottom surface 20, 320 of the body 2. The first integration plate 82, 382 and optional second integration plate 82, 382 each have a top surface 81, 381; a bottom surface 83, 383; opposing lateral sides; opposing anterior portions 41, 341 and posterior portions 51, 351; and a single vertical aperture 61, 361 extending from the top surface 81, 381 to the bottom surface 83, 383 and aligning with the single vertical aperture 60, 360 of the body 2, when present. In the case of a composite implant 1, 101, 101 a, 201, and 301 with one or more integration plates 82, 382, the top surface 81, 381 would be the outer surface or integration surface of the implant 1, 101, 101a, 201, and 301. Preferably, the integration plate 82, 382 should be designed to be compatibly shaped and match the dimensions of the body 2 of the implant 1, 101, 101a, 201, and 301. In a composite implant 1, 101, 101a, 201, and 301, the components may be permanently assembled together.

The integration plate 82, 382 may be attached or affixed to the main body 2 of the implant 1, 101, 101 a, 201, and 301 using any suitable mechanisms known in the art, for example, a reciprocal connector structure (such as a plurality of posts 84, 384 and holes 12, 312 depicted in FIGS. 7 and 8), fasteners (e.g., a pin, screw, bolt, rod, anchor, snap, clasp, clip, clamp, or rivet), compatibly shaped joints, compatibly shaped undercuts, and/or other suitable connectors having different shapes, sizes, and configurations. An adhesive (e.g., cement, glue, polymer, epoxy, solder, and weld) may also be used to further strengthen any connections described in this specification. The top surface 10, 310 or bottom surface 20, 320 may be recessed or inset at a depth D to allow a thickness T of the integration plate 82, 382 to recess within and form a substantially contiguous outer surface (e.g., continuous with the rounded sides of the posterior 50 lateral portions 30) . Recessing the top surface 10, 310 or bottom surface 20, 320 exposes a ridge 11, 311 against which the anterior portion 41, 341, posterior portion 51, 251 or lateral side of the integration plate 82, 382 may be seated and inset into when brought together with the implant 1, 301.

In addition, the implant 1, 101, 101a, 201, and 301 may comprise some or all of the following implant features alone or in combination. The implant 1, 101, 101a, 201, and 301 may include smooth, rounded, or both smooth and rounded lateral sides 30 and posterior-lateral corners. As best shown in FIG. 4B and FIGS. 5A and 5B, the anterior portion 140, 140a may have a tapered nose 142, 142a to facilitate insertion of the implant 101, 101 a. To further facilitate insertion, the implant 101 may have chamfers 106 at the corners of its posterior portion 150. The chamfers 106 prevent the implant 101 from catching upon insertion, risking potential damage such as severed nerves, while still permitting the implant 101 to have a sharp edge 108.

The implant 1, 101, 101a, 201, and 301 may include an opening 90, 190, 190a, 290, 390, for example, in the anterior portion 40, 140, 140a, 240, and 340. The posterior portion 50, 150, 150a, 250, and 350 may have a similarly shaped opening 90, 190, 190a, 290, 390 (not shown). In some aspects, only the anterior portion 40, 140, 140a, 240, and 340 has the opening 90, 190, 190a, 290, 390 while the posterior portion 50 has an alternative opening 92 (which may have a size and shape different from the opening 90, 190, 190a, 290, 390).

The opening 90, 190, 190a, 290, 390 has a number of functions. One function is to facilitate manipulation of the implant 1, 101, 101a, 201, and 301 by the caretaker. Thus, the caretaker may insert a surgical tool into the opening 90, 190, 190a, 290, 390 and, through the engagement between the surgical tool and the opening 90, 190, 190a, 290, 390, manipulate the implant 1, 101, 101 a, 201, and 301. The opening 90, 190, 190a, 290, 390 may be threaded to enhance the engagement. A suitable surgical tool, such as a distractor (not shown), may be selected by one of ordinary skill in the art.

The implant 101, 101a may also have an Implant Holding Feature (IHF) 194, 194a instead of or in addition to the opening 190, 190a. As illustrated in FIGS. 4A and 5A, the IHF 194, 194a is located proximate the opening 190, 190a in the posterior portion 150, 150a. In this particular example, the IHF 194, 194a is a U-shaped notch. Like the opening 190, 190a, the IHF 194, 194a has a number of functions, one of which is to facilitate manipulation of the implant 101, 101 a by the caretaker. Other functions of the opening 190, 190a and the IHF 194, 194a are to increase visibility of the implant 101, 101 a during surgical procedures and to enhance engagement between bone graft material and adjacent bone.

As illustrated in FIG. 4A, the posterior portion 150 of the implant 101 may be substantially flat. Thus, the posterior portion 150 provides a face that can receive impact from a tool, such as a surgical hammer, to force the implant 101 into position.

The implant 1, 101, 101a, 201, and 301 may be provided with a solid rear wall 242. The rear wall 242 may extend the entire width of the implant body and nearly the entire height of the implant body. Thus, the rear wall 242 can essentially close the anterior portion 40, 140, 140a, 240, and 340 of the implant 1, 101, 101a, 201, and 301. The rear wall 242 may offer one or more of several advantages, including reinforcement of the implant 1, 101, 101a, 201, and 301 and improved bone graft containment. In the cervical application, it may be important to prevent bone graft material from entering the spinal canal.

The implant 1, 101, 101a, 201, and 301 may also have a lordotic angle to facilitate alignment. Depending on the type of implant 1, 101, 101a, 201, and 301, one lateral side 30, 130, 130a, 230, and 330 is preferably generally greater in height than the opposing lateral side 30, 130, 130a, 230, and 330 or the anterior portion 40, 140, 140a, 240, and 340 may be generally greater in height than the opposing posterior portion 50, 150, 150a, 250, and 350. Therefore, the implant 1, 101, 101a, 201, and 301 may better compensate for the generally less supportive bone found in certain regions of the vertebral endplate 25. As much as seven to fifteen degrees of lordosis (or more) may be built into the implant 1, 101, 101a, 201, and 301 to help restore cervical balance.

To enhance movement resistance and provide additional stability under spinal loads in the body, the implant 1, 101, and 301 may comprise one or more anti-expulsion edges 8, 108, and 308 that tend to "dig" into the end-plates slightly and help to resist expulsion. The anti-expulsion edges 8, 108, and 308 may be present on the top surface 10, 110, and 310; the bottom surface 20, 120, and 320; or both surfaces of the implant 1, 101, and 301 (or the top surface 81 of the integration plate 82 when present). Each anti-expulsion edge 8, 108, and 308 may protrude above the plane of the top surface 10, 110, and 310 or bottom surface 20, 120, and 320, with the amount of protrusion increasing toward the anterior face 40, 140, and 340 and the highest protrusion height at the anterior-most edge of the top surface 10, 110, and 310 or bottom surface 20, 120, and 320. The anti-expulsion edges 8, 108, and 308 may be sharpened (e.g., the edge comes to a thin, fine point and may be able to penetrate the bone of vertebral endplates 25).

The anti-expulsion edge 8, 108, and 308 may be oriented toward the anterior portion 40, 140, and 340, or the posterior portion 50, 150, and 350, or either of the opposing lateral sides 30, 130, and 330. The orientation of the anti-expulsion edge 8, 108, and 308 may depend on the intended orientation of the implant 1, 101, and 301 when it has been implanted between vertebrae in the patient.

The implant 1, 101, 101a, 201, and 301 may be composed of any suitable biocompatible material. In an exemplary embodiment, the implant 1, 101, 101a, 201, and 301 may be formed of metal. The metal may be coated or not coated. Suitable metals, such as titanium, aluminum, vanadium, tantalum, stainless steel, and alloys thereof, may be selected by one of ordinary skill in the art. In a preferred embodiment, the implant 1, 101, 101a, 201, and 301 includes at least one of titanium, aluminum, and vanadium, without any coatings. In a more preferred embodiment, the implant 1, 101, 101a, 201, and 301 is comprised of titanium or a titanium alloy. An oxide layer may naturally form on a titanium or titanium alloy. Titanium and its alloys are generally preferred for certain embodiments of the present invention due to their acceptable, and desirable, strength and biocompatibility. In this manner, certain embodiments of the present interbody spinal implant 1, 101, 101a, 201, and 301 may have improved structural integrity and may better resist fracture during implantation by impact.

In the case of a composite, the implant 1, 101, 101a, 201, and 301 may further comprise another suitable biocompatible material. For example, in the case of a composite implant 1, 101, 101a, 201, and 301 with one or more integration plates 82, 382, the integration plates 82, 382 may be formed from the metals described above and the body 2 of the implant 1, 101, 101a, 201, and 301 may be formed from a plastic, polymeric, or composite material. For example, suitable polymers may comprise silicones, polyolefins, polyesters, polyethers, polystyrenes, polyurethanes, acrylates, and co-polymers and mixtures thereof. Certain embodiments of the present invention may be comprised of a biocompatible, polymeric matrix reinforced with bioactive fillers, fibers, or both. Certain embodiments of the present invention may be comprised of urethane dimethacrylate (DUDMA)/tri-ethylene glycol dimethacrylate (TEDGMA) blended resin and a plurality of fillers and fibers including bioactive fillers and E-glass fibers. In another embodiment, the body comprises polyetherether-ketone (PEEK), hedrocel, or ultra-high molecular weight polyethylene (UHMWPE). Hedrocel is a composite material composed of carbon and an inert metal, such as tantalum. UHMWPE, also known as high-modulus polyethylene (HMPE) or high-performance polyethylene (HPPE), is a subset of the thermoplastic polyethylene, with a high molecular weight, usually between 2 and 6 million.

EXAMPLE SURGICAL METHODS

The following examples of surgical methods are included to more clearly demonstrate the overall nature of the invention. These examples are exemplary, not restrictive, of the invention.

Certain embodiments of the invention are particularly suited for use during interbody spinal implant procedures currently known in the art. For example, the disc space may be accessed using a standard mini open retroperitoneal laparotomy approach. The center of the disc space is located by AP fluoroscopy taking care to make sure the pedicles are equidistant from the spinous process. The disc space is then incised by making a window in the annulus for insertion of certain embodiments of the spinal implant 1, 101, 101a, 201, and 301 (a 32 or 36 mm window in the annulus is typically suitable for insertion). The process according to the invention minimizes, if it does not eliminate, the cutting of bone. The endplates 25 are cleaned of all cartilage with a curette, however, and a size-specific rasp (or broach) may then be used.

Use of a rasp preferably substantially minimizes or eliminates removal of bone, thus substantially minimizing or eliminating impact to the natural anatomical arch, or concavity, of the vertebral endplate 25 while preserving much of the apophyseal rim. Preservation of the anatomical concavity is particularly advantageous in maintaining biomechanical integrity of the spine. For example, in a healthy spine, the transfer of compressive loads from the vertebrae to the spinal disc is achieved via hoop stresses acting upon the natural arch of the endplate 25. The distribution of forces, and resultant hoop stress, along the natural arch allows the relatively thin shell of subchondral bone to transfer large amounts of load.

During traditional fusion procedures, the vertebral endplate natural arch may be significantly removed due to excessive surface preparation for implant placement and seating. This is especially common where the implant is to be seated near the center of the vertebral endplate 25 or the implant is of relatively small medial-lateral width. Breaching the vertebral endplate natural arch disrupts the biomechanical integrity of the vertebral endplate 25 such that shear stress, rather than hoop stress, acts upon the endplate surface. This redistribution of stresses may result in subsidence of the implant 1, 101, 101a, 201, and 301 into the vertebral body.

Preferred embodiments of the surgical method minimize endplate bone removal on the whole, while still allowing for some removal along the vertebral endplate 25 far lateral edges where the subchondral bone is thickest. Still further, certain embodiments of the interbody spinal implant 1, 101, 101a, 201, and 301 include smooth, rounded, and highly radiused posterior portions and lateral sides which may minimize extraneous bone removal for endplate preparation and reduce localized stress concentrations. Thus, the interbody surgical implant 1, 101, 101a, 201, and 301 and methods of using it are particularly useful in preserving the natural arch of the vertebral endplate and minimizing the chance of implant subsidence.

Because the endplates 25 are spared during the process of inserting the spinal implant 1, 101, 101a, 201, and 301, hoop stress of the inferior and superior endplates is maintained. Spared endplates allow the transfer of axial stress to the apophasis. Endplate flexion allows the bone graft placed in the interior of the spinal implant 1, 101, 101a, 201, and 301 to accept and share stress transmitted from the endplates 25. In addition, spared endplates minimize the concern that BMP might erode the cancellous bone.

The interbody spinal implant 1, 101, 101a, 201, and 301 is durable and can be impacted between the endplates 25 with standard instrumentation. Therefore, certain embodiments of the invention may be used as the final distractor during implantation. In this manner, the disc space may be under-distracted (e.g., distracted to some height less than the height of the interbody spinal implant 1) to facilitate press-fit implantation. Further, certain embodiments of the current invention having a smooth and rounded posterior portion (and lateral sides) may facilitate easier insertion into the disc space. Still further, the surface roughened topography 80, 180, 180a, 280, 380 may lessen the risk of excessive bone removal during distraction as compared to implants having teeth, ridges, or threads currently known in the art even in view of a press-fit surgical distraction method. Nonetheless, once implanted, the interbody surgical implant 1, 101, 101a, 201, and 301 may provide secure seating and prove difficult to remove. Thus, certain embodiments of the interbody spinal implant 1, 101, 101 a, 201, and 301 may maintain a position between the vertebral endplates 25 due, at least in part, to resultant annular tension attributable to press-fit surgical implantation and, post-operatively, improved osteointegration at one or both of the outer surfaces (e.g., top 10 or bottom 20 surfaces).

Surgical implants and methods tension the vertebral annulus via distraction. These embodiments and methods may also restore spinal lordosis, thus improving sagittal and coronal alignment. Implant systems currently known in the art require additional instrumentation, such as distraction plugs, to tension the annulus. These distraction plugs require further tertiary instrumentation, however, to maintain the lordotic correction during actual spinal implant insertion. If tertiary instrumentation is not used, then some amount of lordotic correction may be lost upon distraction plug removal. The interbody spinal implant 1, 101, 101a, 201, and 301, according to certain embodiments of the invention, is particularly advantageous in improving spinal lordosis without the need for tertiary instrumentation, thus reducing the instrument load upon the surgeon. This reduced instrument load may further decrease the complexity, and required steps, of the implantation procedure.

Certain embodiments of the spinal implant 1, 101, 101a, 201, and 301 may also reduce deformities (such as isthmic spondylolythesis) caused by distraction implant methods. Traditional implant systems require secondary or additional instrumentation to maintain the relative position of the vertebrae or distract collapsed disc spaces. In contrast, interbody spinal implant 1, 101, 101a, 201, and 301 may be used as the final distractor and thus maintain the relative position of the vertebrae without the need for secondary instrumentation.

Certain embodiments collectively comprise a family of implants, each having a common design philosophy. These implants and the associated surgical techniques have been designed to address at least the ten, separate challenges associated with the current generation of traditional anterior spinal fusion devices listed above in the Background section of this document.

After desired annulotomy and discectomy, embodiments of the invention first adequately distract the disc space by inserting (through impaction) and removing sequentially larger sizes of very smooth distractors, which have been size matched with the size of the available implant 1, 101, 101a, 201, and 301. Once adequate distraction is achieved, the surgeon prepares the end-plate with a rasp. There is no secondary instrumentation required to keep the disc space distracted while the implant 1, 101, 101a, 201, and 301 is inserted, as the implant 1, 101, 101a, 201, and 301 has sufficient mechanical strength that it is impacted into the disc space. In fact, the height of the implant 1, 101, 101a, 201, and 301 is preferably about 1 mm greater than the height of the rasp used for end-plate preparation, to create some additional tension in the annulus by implantation, which creates a stable implant construct in the disc space.

The implant geometry has features which allow it to be implanted via any one of an anterior, antero-lateral, or lateral approach, providing tremendous intra-operative flexibility of options. The implant 1, 101, 101a, 201, and 301 has adequate strength to allow impact, and the sides of the implant 1, 101, 101a, 201, and 301 may have smooth surfaces to allow for easy implantation and, specifically, to prevent binding of the implant 1, 101, 101a, 201, and 301 to soft tissues during implantation.

The invention encompasses a number of different implant 1, 101, 101a, 201, and 301 configurations, including a composite implant formed of top and optional bottom plates (components), for example, made out of titanium. The integration surfaces exposed to the vertebral body have a roughened surface topography 80, 180, 180a, 280, 380 to allow for bony in-growth over time, and to provide resistance against expulsion. The top and bottom titanium plates may be assembled together with the implant body. The net result is a composite implant that has engineered stiffness for its clinical application. The axial load may be borne by the polymeric component of the construct.

It is believed that an intact vertebral end-plate deflects like a diaphragm under axial compressive loads generated due to physiologic activities. If a spinal fusion implant is inserted in the prepared disc space via a procedure which does not destroy the end-plates, and if the implant contacts the end-plates only peripherally, the central dome of the end-plates can still deflect under physiologic loads. This deflection of the dome can pressurize the bone graft material packed inside the spinal implant, hence allowing it to heal naturally. The implant 1, 101, 101a, 201, and 301 designed according to certain embodiments allows the vertebral end-plate to deflect and allows healing of the bone graft into fusion.

Although illustrated and described above with reference to certain specific embodiments and examples, the present invention is nevertheless not intended to be limited to the details shown. Rather, various modifications may be made in the details within the scope and range of equivalents of the claims and without departing from the spirit of the invention. It is expressly intended, for example, that all ranges broadly recited in this document include within their scope all narrower ranges which fall within the broader ranges. In addition, features of one embodiment may be incorporated into another embodiment.

**Claims**

1. An interbody spinal implant comprising:

   a top surface, a bottom surface, opposing lateral sides, and opposing anterior and posterior portions; and
   at least a portion of the top surface, the bottom surface, or both surfaces includes an integration surface having a roughened surface topography, without sharp teeth, adapted to grip bone through friction generated when the implant is placed between two adjacent vertebrae and to inhibit migration of the implant,
   wherein the integration surface has (a) a plurality of grains; (b) intergranular boundaries between the plurality of grains; and (c) unsatisfied chemical bonds.

2. The interbody spinal implant according to claim 1, wherein the plurality of grains comprise titanium or an alloy of titanium, aluminum, and vanadium.

3. The interbody spinal implant according to claim 1 or 2, wherein the plurality of grains comprise hexagonal crystals.

4. The interbody spinal implant according to any one of claims 1-3, wherein the plurality of grains have an average diameter ranging from about 1-20 $\mu$m, preferably from about 1-5 $\mu$m.

5. The interbody spinal implant according to any one of claims 1-4, wherein the unsatisfied chemical bonds include hydroxylated reactive groups.

6. The interbody spinal implant according to any one of claims 1-5, wherein the unsatisfied chemical bonds are hydrophilic.

7. The interbody spinal implant according to any one of claims 1-6, wherein the integration surface has an outermost surface and a subsurface, and the subsurface comprises a distance of about a single grain diameter or the subsurface penetrates a distance of from about 1-5 $\mu$m.

8. The interbody spinal implant according to any one of claims 1-7, wherein the integration surface has an outermost surface and a subsurface, and the subsurface comprises the plurality of grains with unsatisfied bonds.

9. The interbody spinal implant according to any one of claims 1-8, further comprising a substantially hollow center and a single vertical aperture which (a) extends from the top surface to the bottom surface, (b) has a size and shape predetermined to maximize the surface area of the top surface and the bottom surface available proximate the anterior and posterior portions while maximizing both radiographic visualization and access to the substantially hollow center, and (c) defines a transverse rim.

10. The interbody spinal implant according to any one of claim 1-9, wherein the roughened surface topography comprises macro features, micro features, and nano features.

11. The interbody spinal implant according to claim 10, wherein:

    the macro features have a mean spacing between about 400-2,000 microns, a maximum peak-to-valley height between about 40-500 microns, and an average amplitude between about 20-200 microns;
the micro features have a mean spacing between about 20-400 microns, a maximum peak-to-valley height between about 2-40 microns, and an average amplitude between about 1-20 microns; and
the nano features have a mean spacing between about 0.5-20 microns, a maximum peak-to-valley height between about 0.2-2 microns, and an average amplitude between about 0.01-1 microns.

12. The interbody spinal implant according to any one of claims 1-11, further comprising a first integration plate and optionally a second integration plate recessed into the top surface, the bottom surface, or both surfaces of the implant, wherein the first integration plate, the second integration plate, or both comprise the integration surface.

13. A process for producing an integration surface on an interbody spinal implant having a top surface and a bottom surface where at least a portion of at least one of the top surface and the bottom surface comprises the integration surface having a roughened surface topography, without sharp teeth, adapted to grip bone through friction generated when the implant is placed between two vertebrae and to inhibit migration of the implant, the process comprising:

texturing a surface by chemical processes, mechanical processes, or both processes to provide a plurality of grains and intergranular boundaries between the plurality of grains; and
chemically etching the surface to provide unsatisfied chemical bonds, wherein the plurality of grains and the intergranular boundaries etch at different rates, and optionally aseptically cleaning using plasma or an energy-based system to refine the one or more oxide layers to provide a sterile condition and to preserve properties of the surface.

14. The process according to claim 13 further comprising subsequently cleaning and packaging the interbody spinal implant to preserve the unsatisfied chemical bonds, preferably preserving the interbody spinal implant in an anaerobic environment.

15. The process according to claim 14, wherein the chemical etching produces one or more oxide layers having a thicknesses in a range of 20 to 500 nanometers.

FIG. 1

25

60

80

FIG. 2A

60

80

25

FIG. 2B

FIG. 3A

FIG. 3B

FIG. 4A

FIG. 4B

FIG. 5A

FIG. 5B

FIG. 6

FIG. 7

FIG. 8

| MACRO SURFACE GENERATION | MICRO AND NANO SURFACE GENERATION |
|---|---|

MACHINING → ACID ETCHING (HEAVY) → ALO₂ BLAST → HCL ACID ETCH → CLEANING

## FIG. 9

146852          25852

2

1

141          141

14741

**Ra = Average** (1, 4, 6, 8, 5, 2, 1, 4, 1, 2, 1, 4, 7, 4, 1, 2, 5, 8, 2, 1, 4, 1, 1)

Ra = 3.26

## FIG. 10

Rpm = average(Rp1, Rp2, Rp3, ...)
Rvm = average(Rv1, Rv2, Rv3, ...)
RzDIN = Rtm = average(Rt1, Rt2, Rt3, ...)

## FIG. 11

## FIG. 12

FIG. 13

Sm = average(S$_1$, S$_2$, S$_3$, ...)

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5258098 A
- US 5507815 A
- US 5922029 A
- US 6193762 B